# EUROPEAN PATENT SPECIFICATION

(11) **EP 4 336 968 B1**
(45) Date of publication and mention of the grant of the patent: **09.12.2026**
(21) Application number: 23192284.0
(22) Date of filing: 18.08.2023
(51) Int. Cl.: H05B 3/00, A61N 5/06, F21S 4/28, G05D 23/19

(54) **AN INFRARED LAMP TUBE HEAT DISSIPATION AUTOMATIC CONTROL SYSTEM**
AUTOMATISCHES STEUERSYSTEM FÜR DIE WÄRMEABLEITUNG EINER INFRAROTLAMPENRÖHRE
SYSTÈME DE COMMANDE AUTOMATIQUE DE DISSIPATION DE CHALEUR DE TUBE DE LAMPE INFRAROUGE

(30) Priority: 06.09.2022 TW 111133777
(43) Date of publication of application: 13.03.2024
(73) Proprietor: Liu, Chen Ya, 324 Taoyuan City (TW); Liu, Chin Yuan, 324 Taoyuan City (TW)
(72) Inventor: Liu, Chen Ya, 324 Taoyuan City (TW); Liu, Chin Yuan, 324 Taoyuan City (TW)
(74) Representative: Cabinet Chaillot

(56) References cited:
- TW-U- M 609 075
- TW-U- M 610 319
- US-A- 6 148 909
- US-A1- 2011 277 983

## Description

### BACKGROUND OF THE INVENTION

### 1. Field of the Invention:

An infrared lamp tube heat dissipation automatic control system, especially one that calculates the optimal flow rate of the coolant in a proportional mode that the higher the temperature, the faster the flow rate, and perform contact heat dissipation to the halogen bulb.

### 2. Description of the Related Art:

Infrared rays will penetrate into the depths of the human skin for 40 mm, by promoting the resonance of human cell molecules, charged and movement to expand the blood vessels, making blood circulation smooth and generate self-heating, and also increase the temperature of the deep body and improve metabolism, activate cell tissue, promote the excretion of toxins and wastes in the body and relieves fatigue. When the temperature of the body part increased, it can inhibit the proliferation of cancer cells and activate the immune system. As the body temperature rises with 1°C, the immune system is activated 5 to 6 times. Therefore, by increasing the temperature of the body part, it can relieve pain and prevent the proliferation of cancer cells, and activate the immune system to reduce cancer cells.

Also, the infrared rays are irradiated mostly by the halogen bulbs, to achieve the irradiation effect; however, some halogen bulbs have an operating temperature about 324.4 Celsius degrees, so the heat dissipation of the infrared lamps composed of halogen lamps becomes important issue.

Taiwan Patent No. M610319, An infrared device with fluid heat dissipation, comprising: the body is a long body made of aluminum extrusion, and the section of the body is concave so that the two ends of the body form two concave end surfaces, and an accommodating space is formed inside the body, At least one fluid channel is provided between the concave end faces with the accommodating space as the center, and each halogen lamp is located in the accommodating space, so that the side position of each halogen lamp corresponds to the side position of the fluid channel, forming each There is a barrier between the halogen lamp and the fluid channel, and the filter is statically placed in the accommodating space; thereby, when the halogen lamp emits full-wavelength colored light through the filter, the color temperature of the full-wavelength colored light is changed, and the filter Infrared color light is emitted to the outside of the light guide plate, and is separated from the halogen lamp by a barrier with the fluid in the fluid channel, so that the fluid flows to perform non-contact heat dissipation on the halogen lamp.

However, although having non-contact heat dissipation, the working temperature of the halogen lamp is too high, non-contact heat dissipation for halogen lamps is not easy to achieve the desired heat dissipation effect, and after the high temperature of halogen lamps accumulating for a long time, it will greatly affect the service life and reliability of halogen lamps. Thus, solving the heat dissipation problem on the halogen lamp problem of the infrared lamp tube becomes the subject of this present invention.

US 6 148 909 A discloses a temperature device and temperature comtrol method.

### SUMMARY OF THE INVENTION

A primary objective of the present invention is to provide an infrared lamp tube heat dissipation automatic control system, using the flowing coolant directly contact cool the halogen lamp, and calculates the optimal flow rate of the coolant in a proportional mode that the higher the temperature, the faster the flow rate, so as to achieve the most effective mode of heat dissipation, thereby improving product reliability and the service life.

In order to achieve the above objective, the present invention, according to claim 1, including: an elongated housing, whose section is concave to form an accommodating space, the bottom of the elongated housing is provided with at least one installation hole; at least one lamp holder corresponding to the installation hole arranged at the bottom of the elongated housing; at least one halogen bulb arranged on the lamp holder, and the upper section of the halogen bulb protrudes into the accommodating space; a light-transmitting board arranged on the top of the elongated housing; two left and right covers arranged on the left and right sides of the elongated housing respectively, and a flow hole is provided on the two left and right covers relative to the accommodating space; wherein the infrared lamp tube further includes an automatic control system for heat dissipation, and the automatic control system for heat dissipation includes: a circulation line, which includes an input end and an output end, respectively connected to the flow holes of the right and left covers, for injecting coolant into the accommodating space of the infrared lamp tube and circulating it, so as to cool down the halogen bulb; a temperature sensor is arranged in the infrared lamp tube to detect the temperature of the coolant in the accommodating space; a controller, including a microprocessor and a memory, is electrically connected to the temperature sensor, and calculates the temperature of the coolant; a flow rate sensor, arranged on the circulation line to detect the flow rate of the coolant and transmit the value to the controller; a flow control valve, including a first control valve and a driving unit, the driving unit receives an instruction signal from the controller to control the first control valve to adjust its flow; a pump, installed on the circulation line and electrically connected to the controller, for pumping the coolant to circulate; a cooling device, installed on the circulation line, to lower the temperature of the coolant flowing out from the output end; and according to the temperature of the coolant in the accommodating space and the value provided by the flow rate sensor, the controller calculates the optimal flow rate of the coolant in a proportional mode that the higher the temperature, the faster the flow rate, and then control the flow rate by the flow control valve, so as to achieve predetermined coolant temperature and perform contact heat dissipation to the halogen bulb.

Also, the driving unit of the flow control valve is composed of an electromagnetic solenoid, a micro motor or a piezoelectric unit.

Also, the flow rate sensor and the flow control valve include independent structures or combined structures.

Also, the present invention further includes an external supply liquid entering the cooling device or the circulation line through a supply tube, and the supply tube is provided with a second control valve, the second control valve is electrically connected to the controller.

Also, the left and right covers are further locked on both sides of the elongated housing by multiple screws, and the left and right covers further include a sealing gasket.

Also, the light-transmitting board further includes two joint strips respectively pressed and fixed on the front and rear sides of the light-transmitting board, so that the light-transmitting board is fixed on the top of the elongated housing.

Also, the light-transmitting board is composed of a light guide plate.

Also, in a preferred embodiment, the installation hole of the elongated housing is a screw hole, and the lamp holder includes: a hollow seat, having a ring shaped body formed on the top thereof, a protruding thread body that can be locked in the installation hole is formed on the ring shaped body, a first through hole is formed in the protruding thread body, below the first through hole is connected with a second through hole with a larger diameter, and an internal screw thread is formed at the bottom of the second through hole; an O-ring washer, which is sleeved on the bottom of the outer peripheral edge of the protruding thread body, and is pressed against the bottom edge of the elongated housing by the ring shaped body; a waterproof plug, which is tightly placed in the second through hole, the upper part of the waterproof plug is provided with a receiving hole for the lower part of the halogen bulb to be embedded, and an electrical pin of the halogen bulb protrude out the bottom of the waterproof plug; and an internal nut, which is locked in an internal screw thread at the bottom of the second through hole, for pressing the upper part of the waterproof plug against the bottom edge surface of the first through hole, so that the first through hole forms a waterproof closed state, and the internal nut has a third through hole.

Also, the waterproof plug is made of elastic material, the receiving hole is matched with the shape of the lower part of the halogen bulb, so that it can be combined into a tight fit, and the bottom of the receiving hole is provided with two small perforations for letting the electrical pin of the halogen bulb protrude and locate in the third through hole.

With the features above disclosed, according to the temperature of the coolant in the accommodating space and the value provided by the flow rate sensor, the automatic control system for heat dissipation calculates the optimal flow rate of the coolant in a proportional mode that the higher the temperature, the faster the flow rate, and then control the flow rate by the flow control valve, so as to achieve the most effective mode of heat dissipation, then perform contact heat dissipation to the halogen bulb, so as to solve the problem of the non-contact heat dissipation of halogen bulb of the prior art that cannot achieve the predetermined heat dissipation effect and resulting in the easy damage of halogen bulb; and further improves the product reliability and the service life. Furthermore, the leakproof structure of the lamp holder achieves a completely leakproof function, thereby enhancing product safety.

### BRIEF DESCRIPTION OF THE DRAWINGS

FIG. 1 is a top perspective view of the infrared lamp tube of the present invention;
FIG. 2 is a bottom perspective view of the infrared lamp tube of the present invention;
FIG. 3 is an exploded perspective view of a part structure of the infrared lamp tube of the present invention;
FIG. 4 is a side view of the infrared lamp tube of the present invention;
FIG. 5 is a sectional view along line 5-5 in FIG. 4;
FIG. 6 is a sectional view along line 6-6 in FIG. 5.
FIG. 7 is a schematic diagram illustrating of the structure of an applicable embodiment of the present invention;
FIG. 8 is a curve diagram illustrating the relation of the flow rate and the temperature of the coolant of the present invention;
FIG. 9 is an exploded perspective view of the leakproof structure of the lamp holder of the present invention;
FIG. 10 is a sectional view of the leakproof structure of the lamp holder of the present invention.

### DETAILED DESCRIPTION OF THE PREFERRED EMBODIMENT

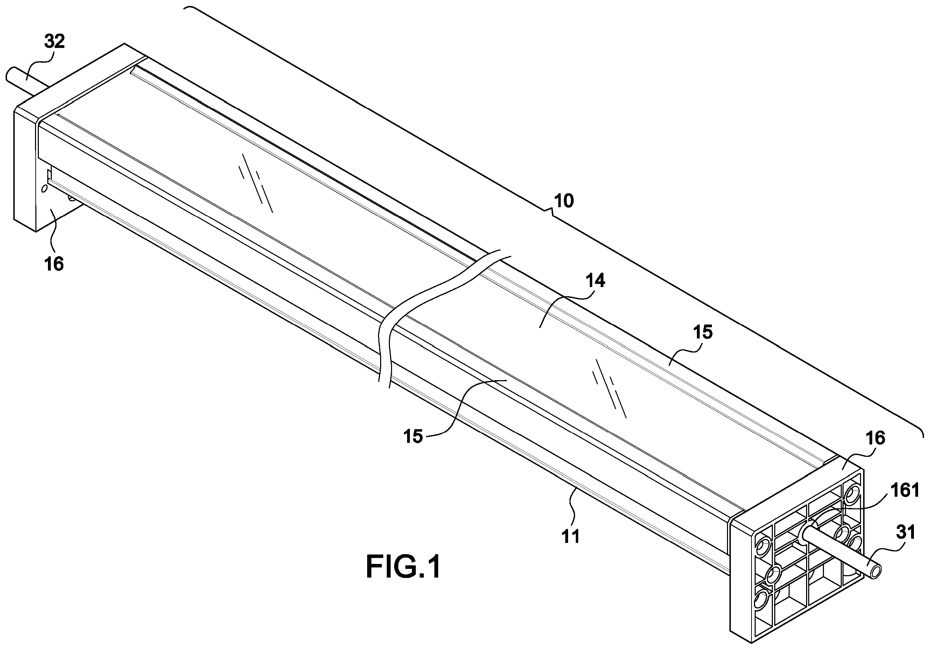

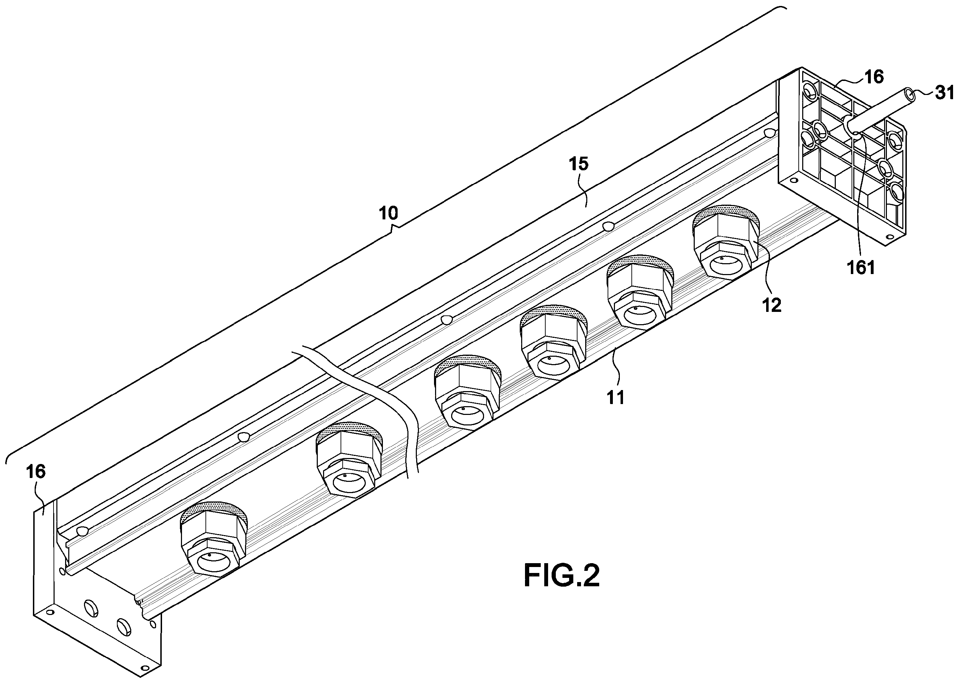

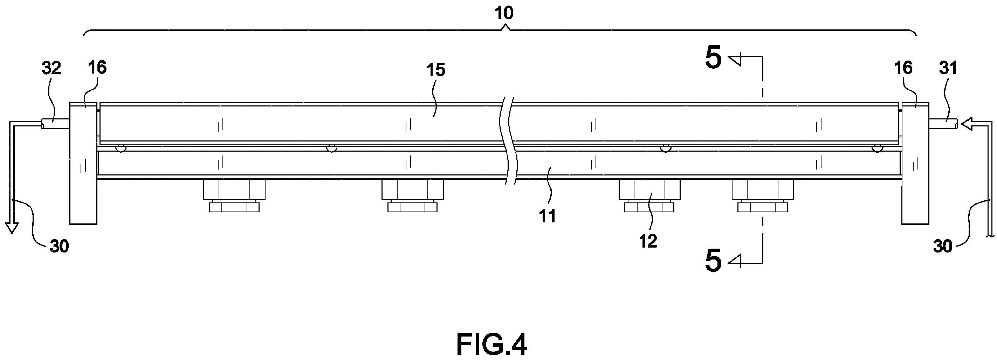

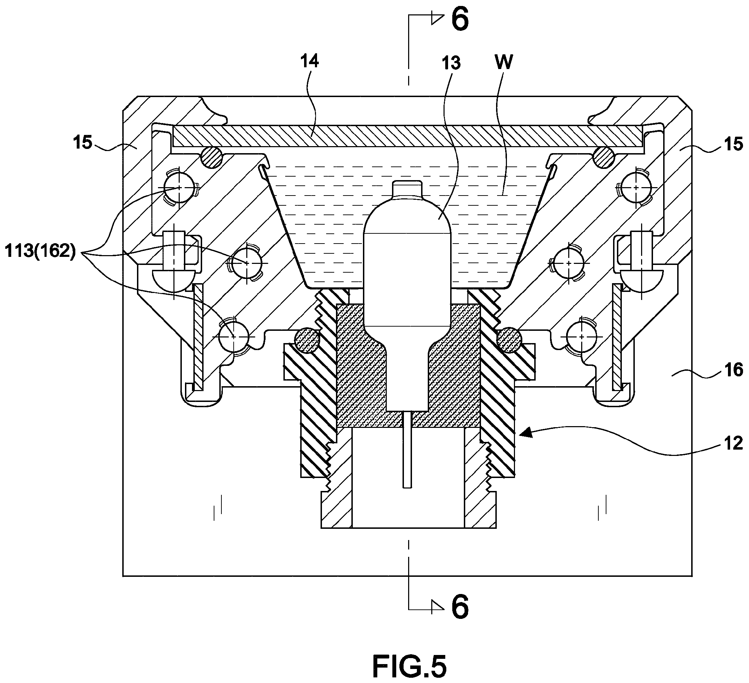

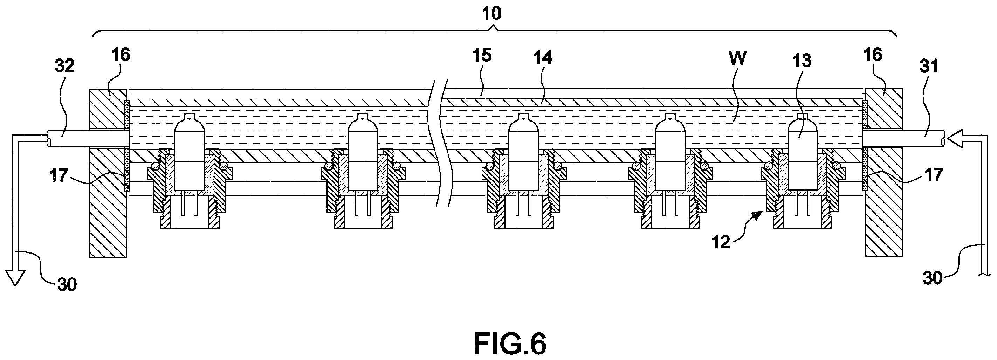

Referring to FIGS. 1-8, the applicable embodiment of the infrared lamp tube heat dissipation automatic control system of the present invention, the infrared lamp tube **10** including: an elongated housing **11,** whose section is concave to form an accommodating space **111,** the bottom of the elongated housing **11** is provided with at least one installation hole **112;** at least one lamp holder **12** corresponding to the installation hole **112** arranged at the bottom of the elongated housing **11;** at least one halogen bulb **13** arranged on the lamp holder **12,** and the upper section of the halogen bulb **13** protrudes into the accommodating space **111.**

A light-transmitting board **14** arranged on the top of the elongated housing **11.** In this embodiment, the light-transmitting board**14** is composed of a light guide plate, includes two joint strips **15** respectively pressed and fixed on the front and rear sides of the light-transmitting board **14,** so that the light-transmitting board **4** is fixed on the top of the elongated housing **11.**

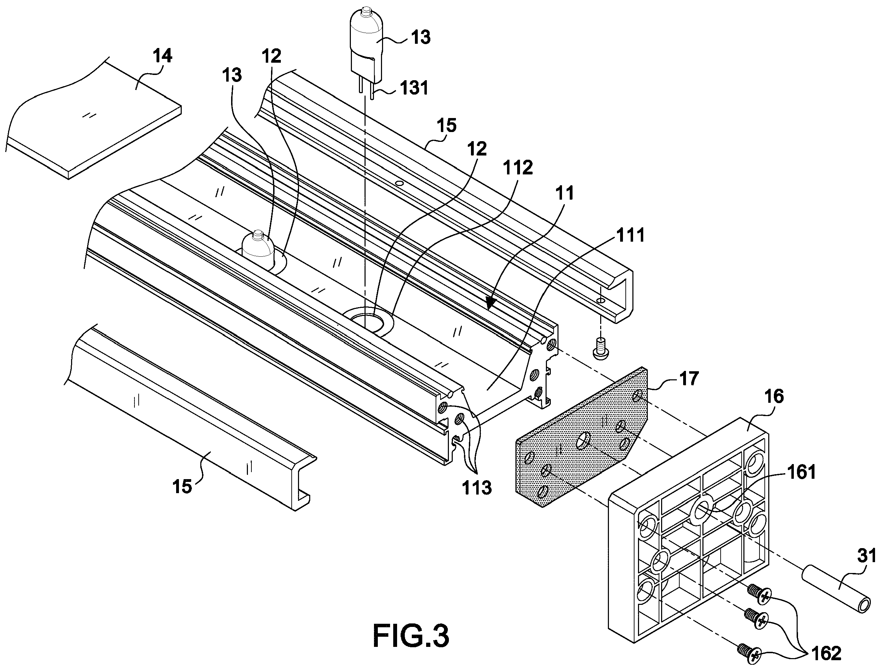

Two left and right covers **16** arranged on the left and right sides of the elongated housing **11** respectively, and a flow hole **161** is provided on the two left and right covers **16** relative to the accommodating space **111;** referring to FIG. 3, in this embodiment, the left and right covers **16** are further locked on a fixing hole **113** of both sides of the elongated housing **11** by multiple screws **162,** and the inner side of the left and right covers **16** further include a sealing gasket **17,** but not limited to this.

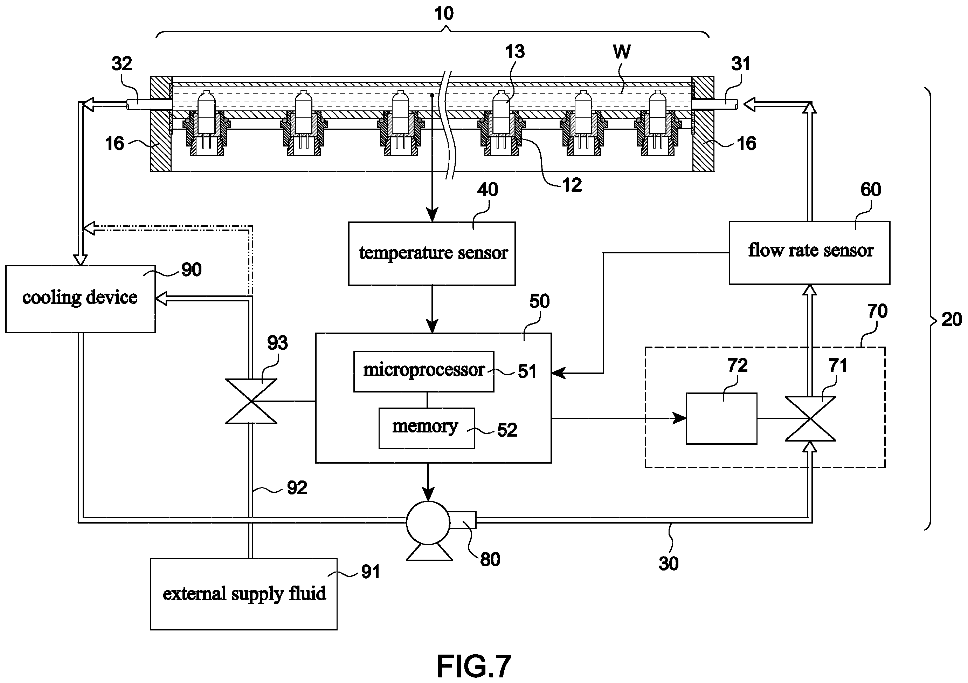

Referring to FIG. 7, the infrared lamp tube **10** further includes an automatic control system for heat dissipation **20,** and the automatic control system for heat dissipation **20** includes:
A circulation line **30,** which includes an input end **31** and an output end **32,** respectively connected to the flow holes **161** of the right and left covers **16,** for injecting coolant **W** into the accommodating space **111** of the infrared lamp tube **10** and circulating it, so as to cool down the halogen bulb **13.**

A temperature sensor **40** is arranged in the infrared lamp tube **10** to detect the temperature of the coolant **W** in the accommodating space **111;** a controller **50,** including a microprocessor **51** and a memory **52,** is electrically connected to the temperature sensor **40,** and calculates the temperature of the coolant **W;**

A flow rate sensor **60,** arranged on the circulation line **30** to detect the flow rate of the coolant **W** and transmit the value to the controller **50;** In this embodiment, the flow rate sensor **60** can be composed of a velocity meter or a flow meter. If using the velocity meter, the flow rate of the coolant **W** can be directly measured; If using flow meter, the controller **50** can calculates the flow rate of the coolant **W** through the cross-sectional area of the circulation line **30;** Such flow rate sensor **60** is already commercially mature products, so the feature of it is not described in detail here.

A flow control valve **70,** including a first control valve **71** and a driving unit **72,** the driving unit **72** receives an instruction signal from the controller **50** to control the first control valve **71** to adjust the flow rate; In this embodiment, the driving unit **72** of the flow control valve **70** is composed of an electromagnetic solenoid, a micro motor or a piezoelectric unit. Such flow control valve **70** is already commercially mature products, so the feature of it is not described in detail here. The flow rate sensor 60 and the flow control valve **70** include independent structures or combined structures.

A pump **80,** installed on the circulation line **30** and electrically connected to the controller **50,** for pumping the coolant **W** to circulate; A cooling device **90,** installed on the circulation line **30,** to lower the temperature of the coolant **W** flowing out from the output end **32;** and in this embodiment, the cooling device **90** includes a heat exchanger.

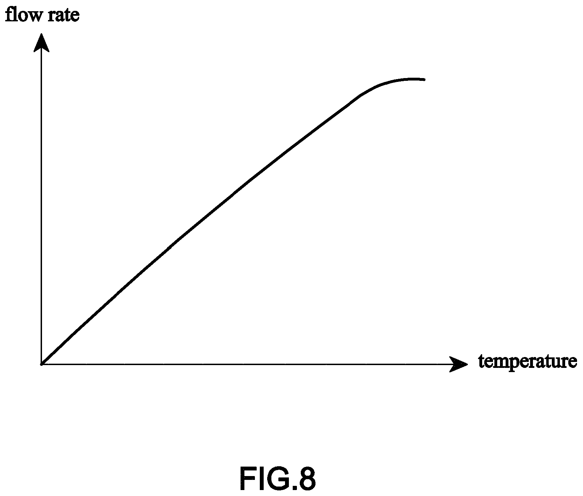

Whereby, according to the temperature of the coolant **W** in the accommodating space **111** and the value provided by the flow rate sensor **60,** as FIG. 8 showing, the controller **50** calculates the optimal flow rate of the coolant **W** in a proportional mode that the higher the temperature, the faster the flow rate, and then control the flow rate by the flow control valve **70,** so as to achieve predetermined coolant **W** temperature and perform contact heat dissipation to the halogen bulb **13.**

Referring to FIG. 7, the present invention further includes an external supply liquid **91** entering the cooling device **90** or the circulation line **30** through a supply tube **92,** and the supply tube **92** is provided with a second control valve **93,** the second control valve 93 is electrically connected to the controller **50.**

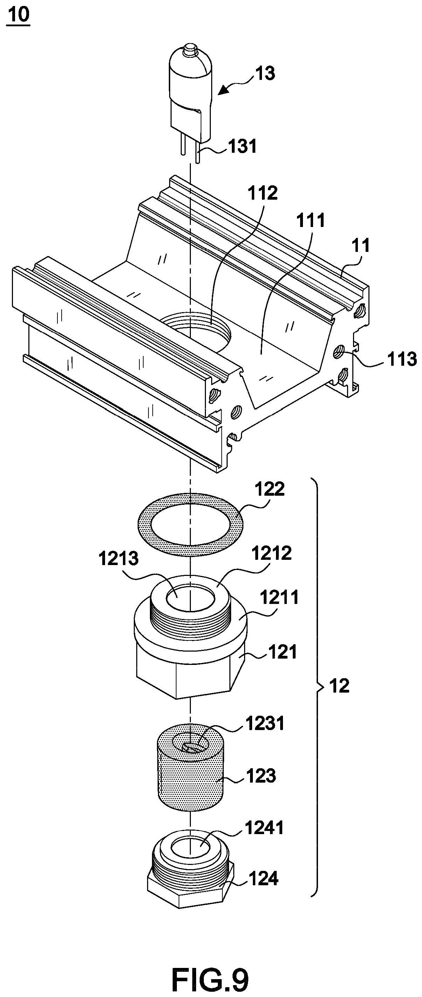

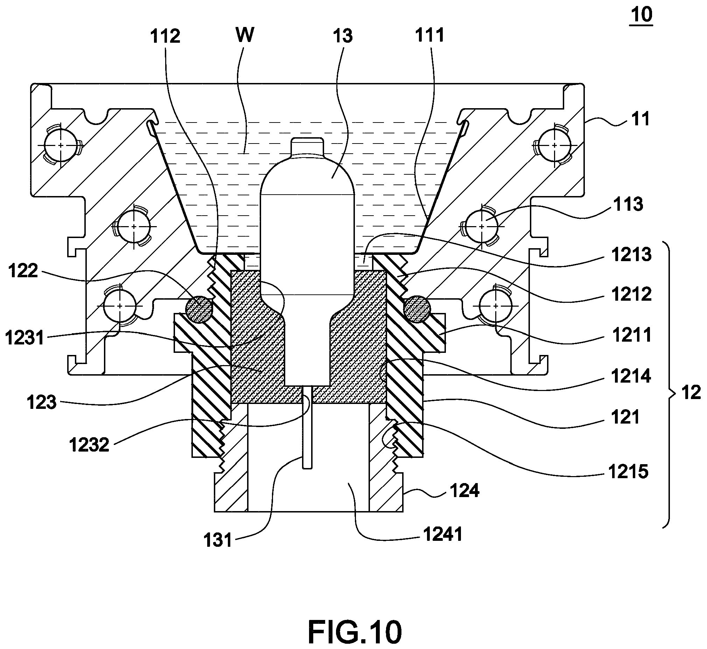

Also, in order to prevent the coolant **W** from leaking caused by the flow rate of the coolant **W** entering and exiting the accommodating space **111,** the present invention further includes a leakproof structure of the lamp holder **12.** Referring to FIGS. 9~10, in an applicable embodiment, the installation hole **112** of the elongated housing **11** is a screw hole, and the lamp holder **12** includes: a hollow seat **121,** having a ring shaped body **1211** formed on the top thereof, a protruding thread body **1212** that can be locked in the installation hole **112** is formed on the ring shaped body **1211,** a first through hole **1213** is formed in the protruding thread body **1212,** below the first through hole **1213** is connected with a second through hole **1214** with a larger diameter, and an internal screw thread **1215** is formed at the bottom of the second through hole **1214;** an O-ring washer **122,** which is sleeved on the bottom of the outer peripheral edge of the protruding thread body **1212,** and is pressed against the bottom edge of the elongated housing **11** by the ring shaped body **1211;** a waterproof plug **123,** which is tightly placed in the second through hole **1214,** the upper part of the waterproof plug **123** is provided with a receiving hole **1231** for the lower part of the halogen bulb **13** to be embedded, and an electrical pin **131** of the halogen bulb **13** protrude out the bottom of the waterproof plug **123;** and an internal nut **124,** which is locked in an internal screw thread **1215** at the bottom of the second through hole **1214,** for pressing the upper part of the waterproof plug **123** against the bottom edge surface of the first through hole **1213,** so that the first through hole **1213** forms a waterproof closed state, and the internal nut **124** has a third through hole **1241.**

In this embodiment, the waterproof plug **123** is made of elastic material, the receiving hole **1231** is matched with the shape of the lower part of the halogen bulb **13,** so that it can be combined into a tight fit, and the bottom of the receiving hole **1231** is provided with two small perforations **1232** for letting the electrical pin **131** of the halogen bulb **13** protrude and locate in the third through hole **1241.**

With the structure disclosed above, first, the lamp holder **12** is combined with the installation hole **112,** and has an excellent sealing effect, which can ensure that the coolant **W** in the accommodating space **111** will not leaks out from the installation hole **112;** moreover, the lower section of the halogen bulb **13** is covered by the waterproof plug **123** in a tight state; in this way, the present invention achieves a complete leakproof structure.

With the features above disclosed, according to the temperature of the coolant **W** in lamp tube and the value provided by the flow rate sensor **60,** the controller **50** calculates the optimal flow rate of the coolant **W** in a proportional mode that the higher the temperature, the faster the flow rate, and then control the flow rate by the flow control valve **70,** so as to achieve predetermined coolant **W** temperature and perform contact heat dissipation to the halogen bulb **13,** thus solving the problem of the non-contact heat dissipation of halogen bulb of the prior art that cannot achieve the predetermined heat dissipation effect and resulting in the easy damage of halogen bulb; and further improves the product reliability and the service life. Furthermore, the leakproof structure of the lamp holder **12** achieves a completely leakproof function, thereby enhancing product safety.

Although particular embodiments of the invention have been described in detail for purposes of illustration, various modifications and enhancements may be made without departing from the scope of the invention as defined by the appended claims.

## Claims

1. An infrared lamp tube heat dissipation automatic control system, including:
an elongated housing **(11),** whose section is concave to form an accommodating space **(111),** the bottom of the elongated housing **(11)** is provided with at least one installation hole **(112),** the installation hole **(112)** of the elongated housing **(11)** is a screw hole;
at least one lamp holder **(12)** corresponding to the installation hole **(112)** arranged at the bottom of the elongated housing **(11);**
at least one halogen bulb **(13)** arranged on the lamp holder **(12),** and the upper section of the halogen bulb **(13)** protrudes into the accommodating space **(111);**
a light-transmitting board **(14)** arranged on the top of the elongated housing **(11);**
two left and right **(16)** covers arranged on the left and right sides of the elongated housing **(11)** respectively, and a flow hole **(161)** is provided on the two left and right covers **(16)** relative to the accommodating space **(111);**
wherein the infrared lamp tube **(10)** further includes an automatic control system for heat dissipation **(20),** and the automatic control system for heat dissipation **(20)** includes:
a circulation line **(30),** which includes an input end **(31)** and an output end **(32),** respectively connected to the flow holes **(161)** of the right and left covers **(16);**
**characterized in that**:
the circulation line (30) is for injecting coolant **(W)** into the accommodating space **(111)** of the infrared lamp tube **(10)** and circulating it, so as to cool down the halogen bulb **(13);**
a temperature sensor **(40)** is arranged in the infrared lamp tube **(10)** to detect the temperature of the coolant **(W)** in the accommodating space **(111);**
a controller **(50),** including a microprocessor **(51)** and a memory **(52),** is electrically connected to the temperature sensor **(40),** and calculates the temperature of the coolant **(W);**
a flow rate sensor **(60),** arranged on the circulation line **(30)** to detect the flow rate of the coolant **(W)** and transmit the value to the controller **(50);**
a flow control valve **(70),** including a first control valve **(71)** and a driving unit **(72),** the driving unit **(72)** receives an instruction signal from the controller **(50)** to control the first control valve **(71)** to adjust its flow;
a pump **(80),** installed on the circulation line **(30)** and electrically connected to the controller **(50),** for pumping the coolant **(W)** to circulate;
a cooling device **(90),** installed on the circulation line **(30),** to lower the temperature of the coolant **(W)** flowing out from the output end **(32);** and
according to the temperature of the coolant **(W)** in the accommodating space **(111)** and the value provided by the flow rate sensor **(60),** the controller **(50)** calculates the optimal flow rate of the coolant **(W)** in a proportional mode that the higher the temperature, the faster the flow rate, and then control the flow rate by the flow control valve **(70),** so as to achieve predetermined coolant **(W)** temperature and perform contact heat dissipation to the halogen bulb **(13),**
and **in that** the lamp holder **(12)** includes:
a hollow seat **(121),** having a ring shaped body **(1211)** formed on the top thereof, a protruding thread body **(1212)** that can be locked in the installation hole **(112)** is formed on the ring shaped body **(1211),** a first through hole **(1213)** is formed in the protruding thread body **(1212),** below the first through hole **(1213)** is connected with a second through hole **(1214)** with a larger diameter, and an internal screw thread **(1215)** is formed at the bottom of the second through hole **(1214);**
an O-ring washer **(122),** which is sleeved on the bottom of the outer peripheral edge of the protruding thread body **(1212),** and pressed against the bottom edge of the elongated housing **(11)** by the ring shaped body **(1211);**
a waterproof plug **(123),** which is tightly placed in the second through hole **(1214),** wherein the upper part of the waterproof plug **(123)** is provided with a receiving hole **(1231)** for the lower part of the halogen bulb **(13)** to be embedded, and an electrical pin **(131)** of the halogen bulb **(13)** protrude out the bottom of the waterproof plug **(13);** and
an internal nut **(124),** which is locked in an internal screw thread at the bottom of the second through hole **(1214),** for pressing the upper part of the waterproof plug **(123)** against the bottom edge surface of the first through hole **(1213),** so that the first through hole forms a waterproof closed state, and the internal nut **(124)** has a third through hole **(1241).**

2. The infrared lamp tube heat dissipation automatic control system as claimed in claim 1, wherein the driving unit **(72)** of the flow control valve **(71)** is composed of an electromagnetic solenoid, a micro motor or a piezoelectric unit.

3. The infrared lamp tube heat dissipation automatic control system as claimed in claim 2, wherein the flow rate sensor **(60)** and the flow control valve **(70)** include independent structures or combined structures.

4. The infrared lamp tube heat dissipation automatic control system as claimed in claim 1, wherein further includes an external supply liquid **(91)** entering the cooling device **(90)** or the circulation line **(30)** through a supply tube **(922),** and the supply tube **(92)** is provided with a second control valve **(93),** the second control valve **(93)** is electrically connected to the controller **(50).**

5. The infrared lamp tube heat dissipation automatic control system as claimed in claim 1, wherein the left and right covers **(16)** are further locked on both sides of the elongated housing **(11)** by multiple screws **(162),** and the left and right **(16)** covers further include a sealing gasket **(17).**

6. The infrared lamp tube heat dissipation automatic control system as claimed in claim 1, wherein the light-transmitting board **(14)** further includes two joint strips **(15)** respectively pressed and fixed on the front and rear sides of the light-transmitting board **(14),** so that the light-transmitting board **(14)** is fixed on the top of the elongated housing **(11).**

7. The infrared lamp tube heat dissipation automatic control system as claimed in claim 1, wherein the light-transmitting board **(14)** is composed of a light guide plate.

8. The infrared lamp tube heat dissipation automatic control system as claimed in claim 1, wherein the waterproof plug **(123)** is made of elastic material, the receiving hole **(1231)** is matched with the shape of the lower part of the halogen bulb **(13),** so that it can be combined into a tight fit, and the bottom of the receiving hole **(1231)** is provided with two small perforations **(1232)** for letting the electrical pin **(131)** of the halogen bulb **(13)** protrude and locate in the third through hole **(1241).**

## Patentansprüche

1. - Automatisches Steuersystem für die Wärmeableitung einer Infrarotlampenröhre, darin eingeschlossen:
ein langgestrecktes Gehäuse (11), dessen Querschnitt konkav ist, um einen Aufnahmeraum (111) zu bilden, wobei der Boden des langgestreckten Gehäuses (11) mit mindestens einer Installationsöffnung (112) versehen ist, wobei die Installationsöffnung (112) des langgestreckten Gehäuses (11) eine Schrauböffnung ist;
mindestens eine Lampenfassung (12), die der am Boden des langgestreckten Gehäuses (11) angeordneten Installationsöffnung (112) entspricht;
mindestens eine Halogenlampe (13), die an der Lampenfassung (12) angeordnet ist, wobei der obere Abschnitt der Halogenlampe (13) in den Aufnahmeraum (111) hervorsteht;
eine lichtdurchlässige Platte (14), die auf der Oberseite des langgestreckten Gehäuses (11) angeordnet ist;
zwei linke und rechte (16) Abdeckungen, die jeweils an der linken und der rechten Seite des langgestreckten Gehäuses (11) angeordnet sind, wobei an den zwei linken und rechten Abdeckungen (16) in Bezug auf den Aufnahmeraum (111) eine Strömungsöffnung (161) vorgesehen ist;
wobei die Infrarotlampenröhre (10) ferner ein automatisches Steuersystem für die Wärmeableitung (20) beinhaltet und das automatische Steuersystem für die Wärmeableitung (20) Folgendes beinhaltet:
eine Kreislaufleitung (30), die ein Eingangsende (31) und ein Ausgangsende (32) beinhaltet, die jeweils mit den Strömungsöffnungen (161) der rechten und linken Abdeckungen (16) verbunden sind;
**dadurch gekennzeichnet, dass**:
die Kreislaufleitung (30) dazu dient, Kühlmittel (W) in den Aufnahmeraum (111) der Infrarotlampenröhre (10) einzuspritzen und umzuwälzen, um die Halogenlampe (13) abzukühlen;
ein Temperatursensor (40) in der Infrarotlampenröhre (10) angeordnet ist, um die Temperatur des Kühlmittels (W) in dem Aufnahmeraum (111) zu erfassen;
eine Steuerung (50), die einen Mikroprozessor (51) und einen Speicher (52) beinhaltet, elektrisch mit dem Temperatursensor (40) verbunden ist und die Temperatur des Kühlmittels (W) berechnet;
ein Durchflusssensor (60) an der Kreislaufleitung (30) angeordnet ist, um die Durchflussrate des Kühlmittels (W) zu erfassen und den Wert an die Steuerung (50) zu übertragen;
ein Durchflussregelventil (70), das ein erstes Regelventil (71) und eine Antriebseinheit (72) beinhaltet, wobei die Antriebseinheit (72) ein Anweisungssignal von der Steuerung (50) empfängt, um das erste Regelventil (71) zur Einstellung seines Durchflusses zu steuern;
eine Pumpe (80), die an der Kreislaufleitung (30) installiert und elektrisch mit der Steuerung (50) verbunden ist, um das Kühlmittel (W) zur Umwälzung zu pumpen;
eine Kühlvorrichtung (90), die an der Kreislaufleitung (30) installiert ist, um die Temperatur des aus dem Ausgangsende (32) ausströmenden Kühlmittels (W) zu senken; und
die Steuerung (50) auf Grundlage der Temperatur des Kühlmittels (W) in dem Aufnahmeraum (111) und des von dem Durchflusssensor (60) bereitgestellten Werts die optimale Durchflussrate des Kühlmittels (W) in einem proportionalen Modus berechnet, bei dem die Durchflussrate umso höher ist, je höher die Temperatur ist, und anschließend die Durchflussrate mittels des Durchflussregelventils (70) steuert, um eine vorbestimmte Temperatur des Kühlmittels (W) zu erreichen und eine Kontaktwärmeableitung an der Halogenlampe (13) durchzuführen,
und dadurch, dass die Lampenfassung (12) Folgendes beinhaltet:
einen hohlen Sitz (121), der einen ringförmigen Körper (1211) aufweist, der an der Oberseite gebildet ist, wobei ein vorstehender Gewindekörper (1212), der in der Installationsöffnung (112) verriegelt werden kann, auf dem ringförmigen Körper (1211) gebildet ist, eine erste Durchgangsöffnung (1213) in dem vorstehenden Gewindekörper (1212) gebildet ist, darunter die erste Durchgangsöffnung (1213) mit einer zweiten Durchgangsöffnung (1214) mit einem größeren Durchmesser verbunden ist, und ein Innenschraubengewinde (1215) am Boden der zweiten Durchgangsöffnung (1214) gebildet ist;
eine O-Ring-Unterlegscheibe (122), die auf den Boden des Außenumfangsrands des vorstehenden Gewindekörpers (1212) aufgesteckt ist und durch den ringförmigen Körper (1211) gegen den unteren Rand des langgestreckten Gehäuses (11) gedrückt wird;
einen wasserdichten Stopfen (123), der dicht in die zweite Durchgangsöffnung (1214) eingesetzt ist, wobei der obere Teil des wasserdichten Stopfens (123) mit einer Aufnahmeöffnung (1231) versehen ist, damit der untere Teil der Halogenlampe (13) darin eingebettet wird und ein elektrischer Stift (131) der Halogenlampe (13) aus dem Boden des wasserdichten Stopfens (13) hervorsteht; und
eine Innenmutter (124), die in einem Innenschraubengewinde am Boden der zweiten Durchgangsöffnung (1214) verriegelt ist, um den oberen Teil des wasserdichten Stopfens (123) gegen die untere Randfläche der ersten Durchgangsöffnung (1213) zu drücken, sodass die erste Durchgangsöffnung einen wasserdicht geschlossenen Zustand bildet, wobei die Innenmutter (124) eine dritte Durchgangsöffnung (1241) aufweist.

2. - Automatisches Steuersystem für die Wärmeableitung einer Infrarotlampenröhre nach Anspruch 1, wobei die Antriebseinheit (72) des Durchflussregelventils (71) aus einem elektromagnetischen Solenoid, einem Mikromotor oder einer piezoelektrischen Einheit besteht.

3. - Automatisches Steuersystem für die Wärmeableitung einer Infrarotlampenröhre nach Anspruch 2, wobei der Durchflusssensor (60) und das Durchflussregelventil (70) unabhängige Strukturen oder kombinierte Strukturen beinhalten.

4. - Automatisches Steuersystem für die Wärmeableitung einer Infrarotlampenröhre nach Anspruch 1, wobei ferner eine externe Zuführflüssigkeit (91) beinhaltet ist, die über ein Zuführrohr (922) in die Kühlvorrichtung (90) oder die Kreislaufleitung (30) eintritt, wobei das Zuführrohr (92) mit einem zweiten Regelventil (93) versehen ist und das zweite Regelventil (93) elektrisch mit der Steuerung (50) verbunden ist.

5. - Automatisches Steuersystem für die Wärmeableitung einer Infrarotlampenröhre nach Anspruch 1, wobei die linke und die rechte Abdeckung (16) ferner mittels mehrerer Schrauben (162) an beiden Seiten des langgestreckten Gehäuses (11) festgeschraubt sind und die linke und die rechte (16) Abdeckung ferner eine Dichtung (17) beinhalten.

6. - Automatisches Steuersystem für die Wärmeableitung einer Infrarotlampenröhre nach Anspruch 1, wobei die lichtdurchlässige Platte (14) ferner zwei Verbindungsleisten (15) beinhaltet, die jeweils an der Vorderseite und der Rückseite der lichtdurchlässigen Platte (14) angedrückt und befestigt sind, sodass die lichtdurchlässige Platte (14) an der Oberseite des langgestreckten Gehäuses (11) befestigt ist.

7. - Automatisches Steuersystem für die Wärmeableitung einer Infrarotlampenröhre nach Anspruch 1, wobei die lichtdurchlässige Platte (14) aus einer Lichtleiterplatte besteht.

8. - Automatisches Steuersystem für die Wärmeableitung einer Infrarotlampenröhre nach Anspruch 1, wobei der wasserdichte Stopfen (123) aus einem elastischen Material hergestellt ist, die Aufnahmeöffnung (1231) an die Form des unteren Teils der Halogenlampe (13) angepasst ist, sodass sie zu einer engen Passung zusammengefügt werden kann, und der Boden der Aufnahmeöffnung (1231) mit zwei kleinen Durchbrechungen (1232) versehen ist, durch die der elektrische Stift (131) der Halogenlampe (13) hervorstehen und sich in der dritten Durchgangsöffnung (1241) befinden kann.

## Revendications

1. - Système de commande automatique de dissipation thermique de tube de lampe infrarouge, comprenant :
un boîtier allongé (11), dont la section est concave pour former un espace de réception (111), la partie inférieure du boîtier allongé (11) comportant au moins un trou de montage (112), le trou de montage (112) du boîtier allongé (11) étant un trou de vis ;
au moins un support de lampe (12) correspondant au trou de montage (112) disposé à la partie inférieure du boîtier allongé (11) ;
au moins une ampoule halogène (13) disposée sur le support de lampe (12), et la partie supérieure de l'ampoule halogène (13) fait saillie dans l'espace de réception (111) ;
une plaque de transmission de lumière (14) disposée sur la partie supérieure du boîtier allongé (11) ;
deux couvercles gauche et droit (16) disposés respectivement sur les côtés gauche et droit du boîtier allongé (11), et un orifice d'écoulement (161) étant prévu sur les deux couvercles gauche et droit (16) par rapport à l'espace de réception (111) ;
le tube de lampe infrarouge (10) comprenant en outre un système de commande automatique pour dissipation thermique (20), et le système de commande automatique pour dissipation thermique (20) comprend :
une conduite de circulation (30), qui comprend une extrémité d'entrée (31) et une extrémité de sortie (32), respectivement reliées aux orifices d'écoulement (161) des couvercles droit et gauche (16) ;
**caractérisé par le fait que** :
la conduite de circulation (30) sert à injecter du réfrigérant (W) dans l'espace de réception (111) du tube de lampe infrarouge (10) et à le faire circuler, de façon à refroidir l'ampoule halogène (13) ;
un capteur de température (40) est disposé dans le tube de lampe infrarouge (10) pour détecter la température du réfrigérant (W) dans l'espace de réception (111) ;
un contrôleur (50), comprenant un microprocesseur (51) et une mémoire (52), est relié électriquement au capteur de température (40) et calcule la température du réfrigérant (W) ;
un capteur de débit (60), disposé sur la conduite de circulation (30) pour détecter le débit du réfrigérant (W) et transmettre la valeur au contrôleur (50) ;
une vanne de régulation de débit (70), comprenant une première vanne de régulation (71) et une unité d'entraînement (72), l'unité d'entraînement (72) recevant un signal d'instruction provenant du contrôleur (50) pour commander la première vanne de régulation (71) afin d'ajuster son débit ;
une pompe (80), installée sur la conduite de circulation (30) et reliée électriquement au contrôleur (50), et destinée à faire circuler le réfrigérant (W) ;
un dispositif de refroidissement (90), installé sur la conduite de circulation (30), pour abaisser la température du réfrigérant (W) sortant par l'extrémité de sortie (32) ; et
en fonction de la température du réfrigérant (W) dans l'espace de réception (111) et de la valeur fournie par le capteur de débit (60), le contrôleur (50) calcule le débit optimal du réfrigérant (W) selon un mode proportionnel, dans lequel plus la température est élevée, plus le débit est rapide, puis régule le débit à l'aide de la vanne de régulation de débit (70), de façon à atteindre une température de réfrigérant (W) prédéterminée et à assurer une dissipation thermique par contact pour l'ampoule halogène (13), et
**par le fait que** le support de lampe (12) comprend :
un siège creux (121), ayant un corps de forme annulaire (1211) formé sur la partie supérieure de celui-ci, un corps fileté en saillie (1212) qui peut être verrouillé dans le trou de montage (112) étant formé sur le corps de forme annulaire (1211), un premier trou traversant (1213) étant formé dans le corps fileté en saillie (1212), au-dessous du premier trou traversant (1213) étant relié un deuxième trou traversant (1214) ayant un plus grand diamètre, et un filetage interne (1215) étant formé à la partie inférieure du deuxième trou traversant (1214) ;
une rondelle torique (122), qui est enfilée sur la partie inférieure du bord périphérique externe du corps fileté en saillie (1212), et pressée contre le bord inférieur du boîtier allongé (11) par le corps de forme annulaire (1211) ;
un bouchon étanche à l'eau (123), qui est placé de manière étanche dans le deuxième trou traversant (1214), la partie supérieure du bouchon étanche à l'eau (123) comportant un trou de réception (1231) destiné à recevoir la partie inférieure de l'ampoule halogène (13), et une broche électrique (131) de l'ampoule halogène (13) faisant saillie hors de la partie inférieure du bouchon étanche à l'eau (13) ; et
un écrou interne (124), qui est verrouillé dans un filetage interne à la partie inférieure du deuxième trou traversant (1214), pour presser la partie supérieure du bouchon étanche à l'eau (123) contre la surface de bord inférieur du premier trou traversant (1213), de telle sorte que le premier trou traversant forme un état fermé étanche à l'eau, et l'écrou interne (124) ayant un troisième trou traversant (1241).

2. - Système de commande automatique de dissipation thermique de tube de lampe infrarouge selon la revendication 1, dans lequel l'unité d'entraînement (72) de la vanne de régulation de débit (71) est composée d'un solénoïde électromagnétique, d'un micromoteur ou d'une unité piézoélectrique.

3. - Système de commande automatique de dissipation thermique de tube de lampe infrarouge selon la revendication 2, dans lequel le capteur de débit (60) et la vanne de régulation de débit (70) comprennent des structures indépendantes ou des structures combinées.

4. - Système de commande automatique de dissipation thermique de tube de lampe infrarouge selon la revendication 1, comprenant en outre un liquide d'alimentation externe (91) entrant dans le dispositif de refroidissement (90) ou la conduite de circulation (30) par l'intermédiaire d'un tube d'alimentation (922), et le tube d'alimentation (92) comporte une seconde vanne de régulation (93), la seconde vanne de régulation (93) étant reliée électriquement au contrôleur (50).

5. - Système de commande automatique de dissipation thermique de tube de lampe infrarouge selon la revendication 1, dans lequel les couvercles gauche et droit (16) sont en outre verrouillés sur les deux côtés du boîtier allongé (11) par de multiples vis (162), et les couvercles gauche et droit (16) comprennent en outre un joint d'étanchéité (17).

6. - Système de commande automatique de dissipation thermique de tube de lampe infrarouge selon la revendication 1, dans lequel la plaque de transmission de lumière (14) comprend en outre deux bandes de jonction (15) respectivement pressées et fixées sur les côtés avant et arrière de la plaque de transmission de lumière (14), de telle sorte que la plaque de transmission de lumière (14) est fixée sur la partie supérieure du boîtier allongé (11).

7. - Système de commande automatique de dissipation thermique de tube à lampe infrarouge selon la revendication 1, dans lequel la plaque de transmission de lumière (14) est composée d'une plaque de guidage de lumière.

8. - Système de contrôle automatique de dissipation thermique de tube à lampe infrarouge selon la revendication 1, dans lequel le bouchon étanche à l'eau (123) est fait de matériau élastique, le trou de réception (1231) est mis en correspondance avec la forme de la partie inférieure de l'ampoule halogène (13), de telle sorte qu'il peut être combiné en un ajustement serré, et la partie inférieure du trou de réception (1231) comporte deux petites perforations (1232) permettant à la broche électrique (131) de l'ampoule halogène (13) de faire saillie et de se positionner dans le troisième trou traversant (1241).
